## Europäisches Patentamt
## European Patent Office
## Office européen des brevets

(19)

(11) Veröffentlichungsnummer: **0 007 287**
**B1**

(12) **EUROPÄISCHE PATENTSCHRIFT**

(45) Veröffentlichungstag der Patentschrift: **11.04.84**

(51) Int. Cl.³: **A 61 F 1/00**

(21) Anmeldenummer: **79810049.1**

(22) Anmeldetag: **14.06.79**

(54) Mit Kohlenstoffasern verstärkter Knochenzement.

(30) Priorität: **29.06.78 EP 78810005**

(43) Veröffentlichungstag der Anmeldung:
**23.01.80 Patentblatt 80/2**

(45) Bekanntmachung des Hinweises auf die Patenterteilung:
**11.04.84 Patentblatt 84/15**

(84) Benannte Vertragsstaaten:
**AT BE IT LU NL SE**

(56) Entgegenhaltungen:
**FR - A - 2 243 915**
**US - A - 4 064 567**

(73) Patentinhaber: **OSTEO AG**
**Bielstrasse 620**
**CH-2545 Selzach (CH)**

(72) Erfinder: **Mittelmeier, Heinz**
**An der Schutzhütte**
**Blieskastel (DE)**
Erfinder: **Moser, Heinz**
**Späretweg 440**
**CH-2545 Selzach (CH)**
Erfinder: **Leu, Beau**
**Hintere Gasse 33**
**CH-2500 Biel (CH)**

(74) Vertreter: **Seehof, Michel**
**c/o AMMANN PATENTANWAELTE AG BERN**
**Schwarztorstrasse 31**
**CH-3001 Bern (CH)**

Courier Press, Leamington Spa, England.

## Mit Kohlenstoffasern verstärkter Knochenzement

Die Erfindung bezieht sich auf einem mit Kohlenstoffasern verstärkten Knochenzement aus autopolymerisierenden Kunststoffen, insbesondere mit Polymethylmethacrylat und Derivaten.

Ein solcher Knochenzement wird in der US—A—4,064,566 beschrieben. Ihm sind Graphitfasern in einer Menge von 2 bis 12 Gewichtsprozenten beigegeben. Durch die Graphitfasern sollten die mechanischen Eigenschaften des Zements verbessert und seine Polymerisationstemperatur gesenkt werden. So lässt sich nach Angabe der US—A—4,064,566 mit einer Graphitfaserbeigabe von 10% die Polymerisationstemperatur auf 52°C senken. Diese Temperatur wird jedoch mit einer Polymerisationszeit von über einer Stunde erkauft. Falls, wie allgemein erforderlich, zwei Prothesenteile eingesetzt werden müssen, ergibt dies eine zusätzliche Operationszeit von fast zwei Stunden, was unter anderem erhöhte Infektionsgefahr bedeutet. Untersuchungen haben überdies gezeigt, dass ein Anteil von Kohlenstoffasern oberhalb von 4 Gewichtsprozenten erhebliche Schwierigkeiten bei der Beimischung und homogenen Verteilung der Fasern bereitet und dass Kohlenstoffaserbeigaben im praktikablen Beimischungsbereich die Polymerisationstemperatur sogar leicht erhöhen und nicht erniedrigen.

Durch die Kohlenstoffaserbeigabe wird auch nicht das bei Knochenzementen bestehende Problem der aseptischen Prothesenlockerung infolge einer durch Abgabe von Restmonomeren und Oligomeren verursachten chemischtoxischen Fremdkörperreaktion am Körpergewebe gelöst, die sich durch Abwehrzellen und Bindegewebe ausdrückt. Sie bewirkt, dass eine direkte chemische Verbindung zwischen den Knochen und der Zementhülle der Prothese nicht zustande kommt. Die Prothese ist also nur mechanisch verankert. Die Verankerung versagt nach bisherigen Erfahrungen jedoch häufig im Laufe der Zeit. In der Folge tritt gewöhnlich eine Prothesenlockerung auf, welche zu erheblichen Beschwerden Anlass gibt und schliesslich zur Prothesenentfernung mit Hinterlassung eines erheblichen, vielfach schmerzhaften Defektes führt. Offenbar kommt es im Körpermilieu auch zu einer Zementalterung mit Abbau der Polymerketten und Reduzierung der anfänglichen Zementfestigkeit.

Demgegenüber liegt der Erfindung die Aufgabe zugrunde, einen mit Kohlenstoffasern verstärkten Knochenzement anzugeben, der ausser der erhöhten mechanischen Festigkeit eine verbesserte Verträglichkeit durch Herabsetzen der Polymerisationstemperatur auch dann aufweist, wenn der Kohlenstoffaseranteil in der Grössenordnung liegt, bei der die Fasern dem Zement ohne Schwierigkeiten beigemischt werden können, dessen chemischtoxische Fremdkörperreaktion vermindert ist und der ein besseres Anwachsen der Knochens an der Zementoberfläche ermöglicht.

Diese Aufgabe wird dadurch gelöst, dass der Knochenzement ausser den Kohlenstoffasern 1 bis 30 Gewichtsprozente Hydroxylapatit in feinkörniger Partikelform enthält.

Dabei kann die Partikelgrösse in einem weiter Bereich zwischen 2 $\mu$m und 3 mm schwanken.

Kohlenstoffasern sind in verschiedenen Ausführungsformen vorhanden und können als Partikeln oder Faservlies vorliegen. Ein bekanntes Verfahren zur Herstellung von Kohlenstoffasern beruht auf der Pyrolisierung von Acrylfasern. Die Faserdicke der Kohlenstoffasern liegt im Bereich von 6 bis 15 $\mu$m. Aus der obenerwähnten US—A—4,064,566 sind weitere Ausführungsformen bekannt.

Durch den Hydroxylapatit — chemisch $Ca_5$ $(PO_4)_3$ $(OH)$ — wird die mechanische Festigkeit, insbesondere die Dauerschwingfestigkeit, des Knochenzements noch weiter erhöht, seine Körperverträglichkeit vergrössert, günstigenfalls derart, dass ein Anwachsen des Knochens an der Zementoberfläche möglich wird, und eine gute Mischbarkeit des Zements erzielt. Durch die feinkörnige Partikelform des die mineralische Kompenente des natürlichen Knochengewebes bildenden Hydroxylapatits wird im Zement eine gewisse Wabenstruktur erreicht. Einesteils ist der Knochen selbst ein Verbundwerkstoff zwischen kollagenen Faserstrukturen und feinstkörnigem Apatit; andererseits werden auch in der Werkstofftechnik wabenförmige Strukturen verwendet, teils um Substanz au sparen, teils um eine ausreichende Festigkeit zu erhalten. Eine grobe Porosität erscheint jedoch nicht sinnvoll, da sie zur Festigkeitsminderung führt. Da der Apatit mit dem Zement offenbar keine chemische Bindung eingeht, entsteht keine Festigkeitsminderung des Zementes durch chemische Beeinflussung. Die Apatitbeimengung führt aber zweifellos dazu, dass Apatitpartikeln auch an der Kontaktfläche des Zements mit den Knochengewebe vorliegen. Auf diese Weise wird die Oberflächentoxizität des Knochenzementes vermindert. Auch kann der Knochen in diesem Bereich mit den Apatitpartikelchen eine Bindung eingehen, welche die Befestigung der Prothese am Knochen verbessert. Ausserdem wird durch den Hydroxylapatit die Polymerisationstemperatur und damit die Gefahr eine Hitzeschädigung vermindert.

Wieviel Gewichtsprozente und welche Partikelgrössen für den Hydroxylapatit innerhalb der angegebenen Bereiche im einzelnen gewählt werden, hängt vom Anwendungszweck, das heisst Einsatzort und Alter des Patienten ab.

Es ist vorteilhaft, wenn dem Knochenzement Röntgenzusätze, beispielsweise Bariumsalze, beigefügt werden, während Zusätze von sonstigen natürlichen Mineralien nützlich sein können.

Es kann sowohl synthetisch hergestellter Apatit als auch Apatit verwendet werden, der aus natürlichen Knochen gewonnen wurde. Es kann ferner zweckmässig sein, ein Gemisch von Kalziumphosphaten beizumengen.

Der Knochenzement mit den Apatitzusätzen kann dem Operateur auf verschiedene Weise zur Verfügung gestellt werden. Es ist denkbar, dass reiner, herkömmlicher Knochenzement mit Kohlenstoffasern genommen wird, welcher mit monomerer Mischflüssigkeit in irgendeinem dem Fall angemessenen Verhältnis vermengt wird oder dass organisches Kunststoffpulver mit den Kohlenstoffasern und der Apatitanteil in einem bereits bestimmten Mischverhältnis vorliegen oder dass beide Anteile in Pulverform getrennt vorliegen und in einem gewollten Verhältnis gemischt werden.

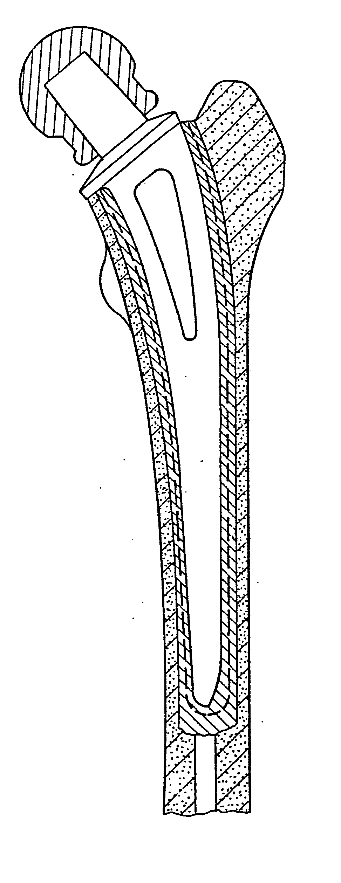

Die einzige Figur der Zeichnung zeigt mit einem Knochenzement nach der Erfindung einzementierte Hüftgelenkprothese.

**Patentansprüche**

1. Mit Kohlenstoffasern verstärkter Knochenzement aus autopolymerisierenden Kunststoffen, insbesondere mit Polymethylmethacrylat und Derivaten, dadurch gekennzeichnet, dass er ausser den Kohlestoffasern 1 bis 30 Gewichtsprozente Hydroxylapatit in feinkörniger Partikelform enthält.

2. Knochenzement nach Anspruch 1, dadurch gekennzeichnet, dass er sowohl synthetisch hergestellten als auch aus natürlichen Knochen gewonnenen Hydroxylapatit enthält.

3. Knochenzement nach Anspruch 1 oder 2, dadurch gekennzeichnet, dass er Röntgenzusätze wie Bariumsalze enthält.

4. Knochenzement nach einem der Ansprüche 1 bis 3, dadurch gekennzeichnet, dass er ein Gemisch von Kalziumphosphaten enthält.

**Revendications**

1. Ciment orthopédique renforcé de fibres de carbone, en matières synthétique autopolymérisante, en particulier avec du polyméthacrilate de méthyle et ses dérivés, caractérisé en ce qu'il contient, en plus des fibres de carbone, 1 à 30 pourcent en poids d'hydroxylapatite en forme de particules à grain fin.

2. Ciment orthopédique selon la revendication 1, caractérisé en ce qu'il contient aussi bien de l'hydroxylapatite fabriquée synthétiquement qu'obtenue de l'os naturel.

3. Ciment orthopédique selon la revendication 1 ou 2, caractérisé en qu'il contient des additifs de radiographie tels que des sels de baryum.

4. Ciment orthopédique selon l'une quelconque des revendications 1 à 3, caractérisé en qu'il contient un mélange de phosphates de calcium.

**Claims**

1. A Carbon fibre-reinforced bone cement formed of autopolymerisable plastics material, particularly with polymethyl methacrylate and its derivatives, characterized in that it contains further to the carbon fibres 1 to 30 percent by weight of hydroxylapatite in finegrained particle form.

2. A bone cement according to Claim 1, characterized in that it contains synthetically produced hydroxylapatite as well as hydroxylapatite obtained from natural bones.

3. A bone cement according to Claim 1 or 2, characterized in that it contains X-ray additives such as barium salts.

4. A bone cement according to any one of the Claims 1 to 3, characterized in that it contains a mixture of calcium phosphates.